# EUROPEAN PATENT APPLICATION

(11) **EP 4 707 772 A1**
(43) Date of publication of application: **11.03.2026**
(21) Application number: 25199970.2
(22) Date of filing: 03.09.2025
(51) Int. Cl.: G01N 3/20, G01N 3/26, G01N 19/04, G01N 33/38

(54) **METHOD FOR TESTING AN ADHESIVE**

(30) Priority: 05.09.2024 IT 202400019864
(71) Applicant: Kerakoll S.p.A., 41049 Sassuolo (MO) (IT)
(72) Inventor: SELMI, Simone, MODENA (IT)
(74) Representative: Puggioli, Tommaso

(57) **Abstract**

A method for testing an adhesive (3) comprises providing a test piece (1) comprising a base slab (2), the adhesive (3) applied to the base slab (2), a tile (4, 4a, 4b), having a first part (4a) adhered to the base slab (2) through the adhesive (3) and a second part (4b) jutting at least from the adhesive (3) and preferably from the base slab (2), the testing method comprises applying a torque moment (Mt) and/or a bending moment (Mf) to the adhesive preferably through the second jutting part (4b) of the tile (4), increasing the applied torque moment (Mt) and/or bending moment (Mf) until breakage of the test piece (1); recording the torque moment (Mt) and/or the bending moment (Mf) that caused the breakage of the test piece (1).[FIG. 4]

## Description

The present invention relates to a method for testing an adhesive, and in particular, a method for testing the behavior and resistance of an adhesive for ceramic and natural stone tiles.

Adhesives for tiles are divided, e.g., based on the UNI EN 12004 standard, into different categories, each characterized by related properties and/or performance.

Generally, the requirements of adhesives for the inclusion thereof in one category or another, and the test methods for testing such requirements are defined by the standards themselves.

Among the optional requirements that characterize adhesives, there is transverse deformation which, however, does not represent a real behavior of the adhesive. Indeed, concisely, transverse deformation is tested on a strip of adhesive, while the actual laid "glued system" is a layered system consisting of various layers from the laying surface, floor or wall, to the tiling.

In this scenario, coating materials and laying substrates are further evolving.

For example, in the market of the former, large-size slabs made of ceramic material, stone materials with a fiberglass mesh or mat are emerging, while in the latter, heating substrates with prefabricated panels made of insulating material, which are not very rigid and not very stable, are becoming widespread.

The main drawback of the known testing methods is that they are increasingly less consistent with the actual working conditions of adhesives, and therefore they could provide parameters that are not significant for the actual performance of the product. For example, in real applications, strains parallel to the laying surface also arise, for example due to differential movements between the layers involved, caused by shrinkage and/or expansions.

The need is felt in the field for a more reliable method for testing adhesives to assess the durability of the "glued system" under all stresses acting thereon.

In this context, a method for testing an adhesive is suggested, which is capable of overcoming at least some of the aforesaid drawbacks of the prior art and meeting the aforesaid need.

In particular, it is the object of the present invention to provide a method for testing an adhesive, in which the adhesive is stressed in a manner more similar to actual usage conditions.

Such an object is achieved by a testing method comprising the technical features set forth in one or more of the appended claims. The dependent claims correspond to possible different embodiments of the invention.

According to an aspect, the present invention relates to a method for testing an adhesive, and in particular, an adhesive for ceramic and natural stone tiles.

The testing method is preferably intended for tests on cementitious adhesives defined, concisely, as a mixture of hydraulic binders, aggregates, and organic additives mixed with water or with a liquid additive added just before use, i.e., for example, class C adhesives according to the UNI EN 12004 standard.

The Applicant observed that, since substrates and coating materials are increasingly flexible and the intended uses are becoming more technical, stresses can occur on the laid system, which act parallel to or outside the laying surface and which act eccentrically on the tiling (thus not with a centered action), resulting, for example, in bending moments (if the stresses act outside the laying surface) or torque moments (if the stresses are parallel to the laying surface, such as in a drivable surface, for example) which also and particularly act on the adhesive.

According to an aspect, the method replicates the aforementioned stresses to test the behavior of the adhesive inserted into a layered system consisting of substrate and coating.

As for the terms and definitions adopted in the present description, reference can also be made to the glossary of the UNI EN 12004 standard.

The method for testing an adhesive comprises providing a test piece comprising at least one base slab defining a laying surface, the adhesive applied to the base slab, a tile, in particular a ceramic or natural stone tile, adhered to the base slab through the adhesive so as to have a first part adhered to the base slab (through the adhesive) and a jutting part at least from the adhesive.

The jutting part protrudes from the adhered part and has a first and a second lateral edge extending therefrom.

The testing method comprises applying a torque moment and/or a bending moment to the adhesive, preferably by means of the jutting part of the tile. The testing method comprises increasing the applied torque moment and/or bending moment until the glued system breaks.

Preferably, the torque moment and/or the bending moment are continuously increased during a test of the adhesive.

Alternatively, the torque moment and/or the bending moment can be increased discretely, step by step, during a test of the adhesive.

Advantageously, the torque moment and/or the bending moment applied to the adhesive, preferably by means of the jutting part of the tile, can stress the adhesive itself both parallel to the laying surface and out of the laying surface, reproducing stresses comparable to those to which the adhesive is actually subjected in modern construction applications. Advantageously, the indicated stress combinations include bending moment stress, torque moment stress, and combined bending and torque moment stress.

Preferably, when the torque moment and the bending moment are applied in a combined manner, the test piece is stressed to a preset torque moment value, e.g., 1 kN, and the bending moment is increased until the test piece itself breaks.

Preferably, applying a torque moment comprises applying to the jutting part a force parallel to the laying surface.

Preferably, the parallel force is applied to the first edge or the second edge.

Preferably, applying a bending moment comprises applying to the jutting part a force orthogonal to the laying surface.

Advantageously, the jutting part defines an arm by means of which the force is transmitted to the adhesive, resulting in a moment thereon. Moment means a force or a summation of forces applied with an arm, i.e., a distance between the point of application and the point of stress.

In order to apply the torque moment to the adhesive, the force can also be applied to the first adhered part, and the arm is shorter than the arm that is present when the force is applied to the jutting part.

Preferably, the method comprises gluing to the jutting part of the tile, on the side opposite to the base slab, a metal plate to which at least the bending moment and/or at least the force orthogonal to the laying surface is applied.

The force orthogonal to the laying surface can be applied according to an entering direction or an exiting direction from the laying surface. Advantageously, the metal plate can make the application of the moment to the adhesive or of a force to the jutting part of the tile more practical. Preferably, providing the test piece comprises providing the base slab, applying the adhesive to the base slab, preferably by means of a toothed spatula, applying the tile to the adhesive, applying to the tile, at least at the adhesive, a force orthogonal to the laying surface.

Preferably, the testing method comprises aging the test piece.

According to an aspect, the present invention relates to a test piece preferably intended for a method for testing an adhesive according to the preceding aspect.

The test piece comprises at least one base slab defining a laying surface, an adhesive applied to the base slab, a tile, preferably made of a ceramic or natural stone, attached to the base slab through the adhesive so as to have a first part adhered to the base slab through the adhesive and preferably to the base slab.

The jutting part of the tile protrudes from the adhered part and has a first and a second lateral edge extending from said adhered part and beyond it. Preferably, the base slab is cementitious.

Preferably, the adhesive is cementitious.

Preferably, the jutting part and the adhered part have the same surface.

Further features and advantages of the present solution will become more apparent from the indicative, and therefore non-limiting, description of at least one preferred but not exclusive embodiment of a method for testing an adhesive and a test piece for a method for testing an adhesive.

Such a description will be given below with reference to the accompanying drawings, provided for indicative and thus non-limiting purposes only, in which:
- figure 1 shows a diagrammatic perspective view of a test piece for a method for testing an adhesive according to the description;
- figure 2 shows a diagrammatic perspective view of a test piece for a method for testing an adhesive according to the description;
- figure 3 shows a diagrammatic side view of the test piece in figure 2 in a step of the testing method according to the description;
- figure 4 shows a top plan view of a test piece for a method for testing an adhesive in a step of the testing method according to the description;
- figure 5 shows a diagrammatic perspective view of a test piece for a method for testing an adhesive in a step of the testing method according to the description.

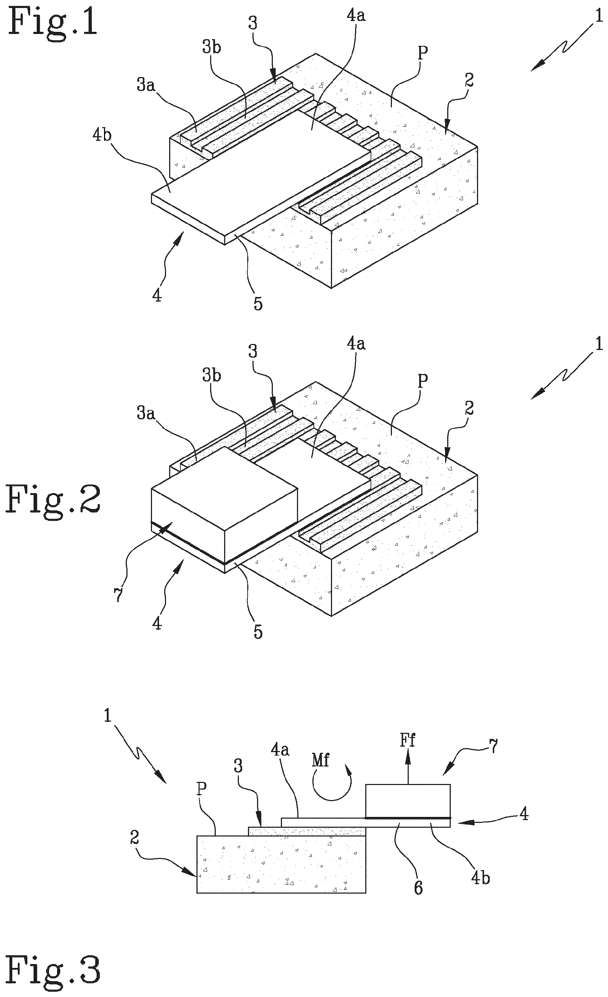

With particular reference to figure 1, reference numeral 1 indicates a test piece according to the present invention.

The test piece 1 comprises a base slab 2 which defines a laying surface P.

For example, the base slab 1 is of a cementitious nature, preferably of the type described in the UNI EN12004 standard.

The test piece 1 comprises an adhesive 3 applied to the base slab 2.

The adhesive 3 is preferably an adhesive for ceramic and natural stone tiles.

The adhesive 3 is, for example, a cementitious adhesive, or an adhesive of class C according to the UNI EN 12004 standard.

The adhesive 3 is preferably cementitious (C), reactive (R), and in dispersion (D) according to the EN 12004 standard.

Advantageously, the testing method according to the present description can be used to test any adhesive 3 which must be used in the test piece 1, as will be better clarified below.

In particular, the testing method allows testing adhesives for ceramic and natural stone tiles, for both floor and wall applications.

Note that in the accompanying diagrammatic figures, the size of the test piece 1 is not necessarily to scale but is functional to the present description.

The test piece 1 comprises a tile 4 attached to the base slab 2 through the adhesive 3.

The tile 4 is preferably made of ceramic or natural stone.

The tile 4 has a first part 4a adhered to the base slab 2 through the adhesive 3 and a second part 4b jutting at least from the adhesive and preferably from the base slab 2, as shown in the figures.

The second jutting part 4b protrudes or extends from the first part 4a adhered beyond the base slab 2.

Preferably, the first part 4a and the second part 4b have the same area.

For example, the tile 4 may be a ceramic tile obtained by pressing according to the UNI EN 14411 standard of the Bia type, fully vitrified and with a water absorption less than or equal to 0.5%, unglazed (UGL), with a planar contact surface with the adhesive and size of 50 +/-1 mm x 100 +/-1 mm (width by length).

The second jutting part 4b has a first and a second lateral edge 5, 6 extending from the first adhered part 4a.

Reference numerals 5 and 6 indicate, in practice, the portions of the edges of tile 4 at the second jutting part 4b thereof.

With reference to figures 2 and 3, the test piece 1 comprises a plate 7, e.g., made of metal, glued to the second part 4b of the tile 4 on the opposite side of the base slab 2 with respect to the tile 4 itself.

A method for testing the adhesive 3 comprises providing a test piece 1 described above.

Preferably, providing the test piece 1 comprises providing the base slab 2 and applying the adhesive 3 to the base slab 2.

Preferably, applying the adhesive to the base slab 2 comprises applying the adhesive 3 with a toothed spatula (not shown) so that the adhesive 3 has a sequence of ridges 3a and grooves 3b.

For example, a toothed spatula with 6x6 mm teeth can be used.

The adhesive 3 can be mixed according to the UNI EN12004 standard, paragraph 6.

Providing the test piece 1 comprises applying the tile 4 to the adhesive 3. Preferably, providing the test piece 1 comprises applying the tile 4 to the adhesive 3 after a time t3, e.g., 5 minutes, from the application of the adhesive 3 to the base slab 2.

The tile 4 is positioned over the adhesive 3 so that the adhesive wets the first part 4a of the tile 4, while the second part 4b remains jutting.

In a preferred example, the first part 4a is equal to the second part 4b of the tile 4.

More precisely, the first part 4a has the same shape and the same size as the second part 4b of the tile 4.

The first part 4a and the second part 4b can be, for example, two squares with a side of 5, i.e., the first and second edges 5 and 6 measure 5 cm.

In order to conveniently apply the tile 4 to the adhesive 3, a template (not shown) can be used.

Providing the test piece 1 preferably comprises applying to the tile 4, at least at the adhesive 3, a force F8, preferably orthogonal to the laying surface P, for a time t8 so as to ensure an adequate pressure onto the tile 4 and a suitable transfer of the adhesive 3.

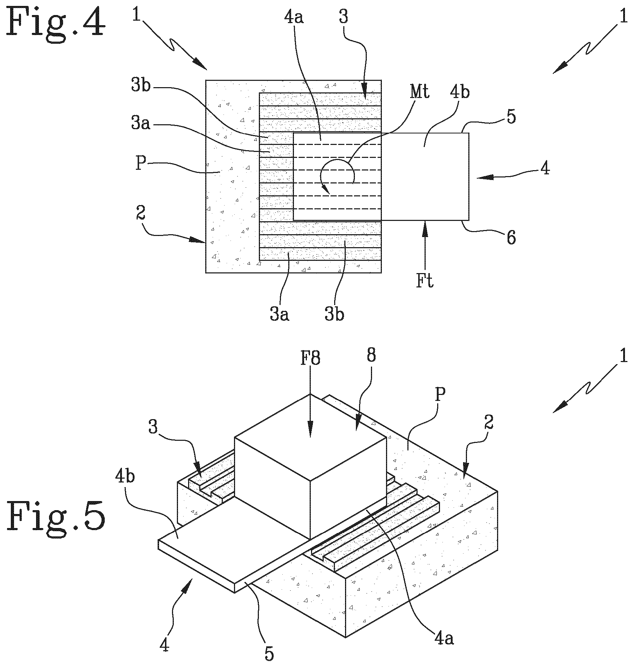

In the example shown in figure 5, applying a force F8 to the tile 4 comprises positioning a weight 8 onto the first part 4a of the tile 4.

For example, the weight 8 is a weight of 20 +/- 2 N, t8 is 30 s, and the contact surface of the weight 8 with the tile 4 is 50x50 mm.

Preferably, providing the test piece 1 comprises aging the test piece 1 itself, i.e., storing the test piece under certain environmental conditions for a determined aging time ts so that it acquires optimal characteristics for use.

For example, the test piece 1 can be aged for 28 days in an environment in which the following conditions are present:
Temperature: 23 +/- 2 °C
Relative humidity: 50 +/- 2 %
Air velocity: < 0.2 m/s

The testing method according to the description comprises applying a torque moment Mt to the adhesive 3, shown in a counterclockwise direction in figure 4.

The torque moment Mt can be applied indifferently either clockwise or counterclockwise.

The torque moment is preferably applied to the adhesive 3 by means of the second jutting part 4b of the tile 4.

Preferably, applying the torque moment Mt comprises applying to the tile 4 a force Ft parallel to the laying surface.

Preferably, the force Ft is applied to the second part 4b of the tile, e.g., to the first or second edge 5, 6 thereof.

Preferably, the force Ft is orthogonal to the first and second edges 5, 6. The torque moment Mt applied to the adhesive 3 depends on the arm of the force Ft with respect to the adhesive 3 itself.

The testing method according to the description comprises applying a bending moment Mf to the adhesive 3, shown in a counterclockwise direction in figure 3.

The bending moment Mf can be applied indifferently either clockwise or counterclockwise.

The bending moment is applied to the adhesive 3 by means of the second jutting part 4b of the tile 4.

Preferably, applying the bending moment Mf comprises applying to the second part 4b of the tile 4 a force Ff orthogonal to the laying surface P. Preferably, the force Ff is orthogonal to the laying surface P.

The bending moment Mf applied to the adhesive 3 depends on the arm of the force Ff with respect to the adhesive 3 itself.

According to the present description, the testing method determines an indirect stress of the adhesive through the application of a bending and/or torque moment to the jutting part of the tile.

Preferably, the force Ff is applied to the tile 4 by means of the metal plate 7.

For example, a dynamometer configured to perform a pull-out test can be used, acting with a tensile force on the plate 7 previously glued to the surface of the tile 4 to be removed.

In such a case, providing the test piece 1 comprises gluing, e.g., with a two-component adhesive, the metal plate 7 to the second jutting part 4b. The torque moment Mt and the bending moment Mf can be applied simultaneously or separately, said bending moment being applied by means of said metal plate.

The force Ft and/or the force Ff can also result from multiple forces applied to the test piece 1.

The testing method comprises increasing the applied torque moment Mt and/or bending moment Mf until the test piece 1 breaks.

The torque moment Mt and/or the bending moment Mf can be increased continuously until breakage or discretely, allowing a more accurate assessment of the features of the adhesive.

In an embodiment, the testing method can comprise applying the torque moment Mt without causing breakage of the test piece 1 in order to pretension it, then applying the bending moment Mf.

Preferably, the method comprises applying a stress parallel to the laying surface P to the first or second edge 5, 6 of the tile 4 so as to generate the torque moment Mt (resulting from the applied eccentric shear stress, with respect to the adhesive 3) without causing breakage of the test piece 1, pretensioning it up to a preset value of the applied force Ft, e.g., 1 kN. The method then comprises applying a stress orthogonal to the laying surface P, i.e., the force Ff at the second jutting part 4b of the tile with an appropriate tool to generate the bending moment Mf.

The test conditions are preferably consistent and defined.

For example, the environmental conditions in which the tests are performed can be: temperature: 23 +/- 2 °C, relative humidity: 50 +/- 2 %, air velocity: < 0.2 m/s.

Preferably, the materials used must be brought to the test conditions at least 24h before performing the test.

Preferably, the tests are performed at the end of aging.

The testing method comprises recording the torque moment Mt and/or the bending moment Mf which caused the breakage of the test piece 1. Recording the torque moment Mt and/or the bending moment Mf which caused the breakage of the test piece 1 can comprise recording the force Ff and/or the force Ft required for the test piece 1 to break.

Preferably, the forces are measured in N. The value of the forces Ft and Ff for the respective arm due to the second jutting part 4b of the tile 4 provide the torque moment Mt and/or the bending moment Mf to which the test piece 1 has been subjected; the bending Mf and torque Mt moments are expressible in Nmm and therefore in Kgm.

Preferably, a torque moment and/or bending moment resistance of the adhesive 3 is determined by an average value calculated on a plurality of test pieces 1, e.g., 10, subjected to testing.

## Claims

1. A method for testing an adhesive (3), said method comprising providing a test piece (1) comprising a base slab (2) defining a laying surface (P), the adhesive (3) applied on the base slab (2), a tile (4, 4a, 4b), preferably made of ceramic or natural stone, having a first part (4a) adhered to the base slab (2) through the adhesive (3) and a second part (4b) jutting at least from the adhesive (3) and preferably from the base slab (2), said second jutting part (4b) having a first and a second lateral edge (5, 6) extending from said first part (4a), said testing method comprising applying a torque moment (Mt) and/or a bending moment (Mf) to the adhesive preferably through said second jutting part (4b) of the tile (4), increasing the applied torque moment (Mt) and/or bending moment (Mf) until breakage of the test piece (1);
recording the torque moment (Mt) and/or the bending moment (Mf) that caused the breakage of the test piece (1).

2. The testing method according to claim 1, wherein applying a torque moment (Mt) comprises applying to said first edge (5) or said second edge (6) of the second jutting part (4b) a force (Ft) parallel to the laying surface (P).

3. The testing method according to claim 1 or 2, wherein applying a bending moment (Mf) comprises applying to said second jutting part (4b) a force (Ff) orthogonal to the laying surface (P).

4. The testing method according to any one of the preceding claims, comprising gluing a plate (7), preferably made of metal, to said second jutting part (4b) on the opposite side with respect to said base slab (2), said bending moment (Mf) being applied by means of said plate (7).

5. The testing method according to claims 3 and 4, wherein said orthogonal force (Ff) is applied by means of said plate (7).

6. The testing method according to any one of the preceding claims, wherein providing the test piece (1) comprises providing the base slab (2), applying the adhesive (3) to the base slab (2), applying the tile (4) to the adhesive (3) in a jutting manner, applying to the tile (4), at least at the adhesive (3), a force (F8) orthogonal to the laying surface (P).

7. The testing method according to any one of the preceding claims, comprising ageing the test piece (1).

8. The testing method according to any one of the preceding claims, wherein applying the adhesive (3) to the base slab (2) comprises applying the adhesive with a toothed spatula.

9. The testing method according to any one of the preceding claims, comprising applying the torque moment (Mt) without breaking the test piece (1) to preload the test piece (1) and applying the bending moment (Mf) until the test piece (1) breaks.

10. The testing method according to any one of claims 1 to 9, wherein the base slab (2) is cementitious.

11. The testing method according to any one of claims 1 to 10, wherein the adhesive (3) is cementitious and/or reactive and/or in dispersion (D) and/or according to standard EN12004.

12. The testing method according to any one of claims 1 to 11, wherein the second jutting part (4b) and the first adhered part (4a) have the same surface and/or the same shape.

13. A test piece for a method for testing an adhesive (3), said test piece comprising:
a base slab (2) defining a laying surface (P);
the adhesive (3) applied to the base slab (2);
a tile (4) attached to the base slab (2) through the adhesive (3) so as to have a first part (4a) adhered to the base slab (2) through the adhesive (3), and a second part (4b) jutting at least from the adhesive (3) and preferably from the base slab (2), said second jutting part (4b) protruding from said first adhered part (4a) and having a first and a second lateral edge (5, 6) extending from said first adhered part (4a).

14. The test piece according to claim 13, wherein the base slab (2) is cementitious.

15. The test piece according to any one of claims 13 or 14, wherein the adhesive (3) is cementitious and/or reactive and/or in dispersion (D) and/or according to standard EN12004.

16. The test piece according to any one of claims 13 to 16, wherein the second jutting part (4b) and the first adhered part (4a) have the same surface and/or the same shape.

17. The test piece according to any one of claims 13 to 16, for a method for testing an adhesive according to any one of claims 1 to 12.
